# EUROPEAN PATENT SPECIFICATION

(11) **EP 2 101 673 B1**
(45) Date of publication and mention of the grant of the patent: **24.01.2018**
(21) Application number: 07869190.4
(22) Date of filing: 13.12.2007
(51) Int. Cl.: A61B 46/10

(54) **MICROSCOPE DRAPE LENS COVER SYSTEM AND ASSEMBLY METHOD**
OBJEKTIVABDECKUNGSSYSTEM FÜR MIKROSKOPABDECKUNG UND MONTAGEVERFAHREN
SYSTÈME DE COUVRE-OBJECTIF DRAPÉ DE MICROSCOPE ET PROCÉDÉ D'ASSEMBLAGE

(30) Priority: 13.12.2006 US 874985 P; 12.12.2007 US 954524
(43) Date of publication of application: 23.09.2009
(73) Proprietor: Microtek Medical, Inc., Columbus, MS 39704 (US)
(72) Inventor: DILLON, Mark S., Columbus, MS 39702 (US); PACK-WALDEN, Ginger C., Columbus, MS 39702 (US); ADAMS, Tammy C., Columbus, MS 39702 (US); DING, Youzhen, Alpharetta, GA 30022 (US)
(74) Representative: Godemeyer Blum Lenze Patentanwälte Partnerschaft mbB - werkpatent
(86) International application number: PCT/US2007/087326
(87) International publication number: WO 2008/076777

(56) References cited:
- EP-A- 0 133 753
- EP-A- 0 941 706
- US-A- 4 385 812
- US-A- 5 608 574
- US-A1- 2005 088 763
- US-A1- 2005 094 269

## Description

### TECHNICAL FIELD OF THE INVENTION

The present invention relates generally to the field of medical drapes and, more particularly, to a microscope drape lens cover system and assembly method.

### BACKGROUND OF THE INVENTION

To minimize the risk of infection to surgical patients in an operating room or to protect medical equipment from a surgical field during surgery, drapes are often utilized. Drapes may be placed over a patient and/or medical equipment to form a sterile barrier, keeping any microorganisms and contaminants that may cause infections from migrating to and from exposed tissue and open wounds. For example, bodily fluids during surgery may settle on medical equipment, which then become contaminated and hazardous to those persons who must work with the equipment. Instead, the bodily fluids will ultimately settle on the drapes and not on the draped medical equipment.

The advancement of medical procedures has correspondingly created a demand for more advanced medical equipment. For example, the surgical microscope has become an integral part of an operating room. The surgical microscope can be ceiling mounted, wall mounted or mounted on a floor stand and typically may be raised or lowered and positioned over any part of a patient's body. The surgical microscope often has multiple eyepieces that permit the surgeon and others to simultaneously view the magnified area under the microscope's objective lens.

A microscope drape, used to create a sterile barrier, may be affixed to the microscope at the lens housing of the objective lens to orient the drape with respect to the remaining structure of the microscope. Other portions of the drape may be spread and positioned to cover the remainder of the microscope structure. In order to be able to protect the objective lens and still be able to see the surgical area, a transparent protective lens is usually associated with the device that couples the drape to the microscope. The transparent protective lens, depending on its positioning, may cause an undesirable glare to the user of the microscope, which may complicate the surgical procedure. US 2005/0094269 A1 discloses the features of the preamble of claim 1.

### SUMMARY OF THE INVENTION

It is an object of the present invention to provide a microscope drape system, a microscope drape coupling system, and a method of coupling a drape to a microscope. This object can be achieved by the features as defined in the independent claims. Further enhancements are characterized in the dependent claims.

Other technical advantages are readily apparent to one skilled in the art from the following figures, descriptions, and claims.

### BRIEF DESCRIPTION OF THE DRAWINGS

For a more complete understanding of the invention, and for further features and advantages, reference is now made, by way of example, to the following description, taken in conjunction with the accompanying drawings, in which:
FIGURE 1 is a perspective view of a microscope drape coupled to a microscope using a drape lens cover system in accordance with one embodiment of the present invention;
FIGURE 2 is an exploded, perspective view of a microscope drape lens cover system in accordance with one embodiment of the present invention;
FIGURE 3 is a cross-sectional view illustrating the microscope drape lens cover system of FIGURE 2 coupled to a microscope in accordance with one embodiment of the present invention; and
FIGURE 4 is a cross-sectional view illustrating a microscope drape coupler in accordance with one embodiment of the present invention in accordance with another embodiment of the present invention.

### DETAILED DESCRIPTION OF EXAMPLE EMBODIMENTS OF THE INVENTION

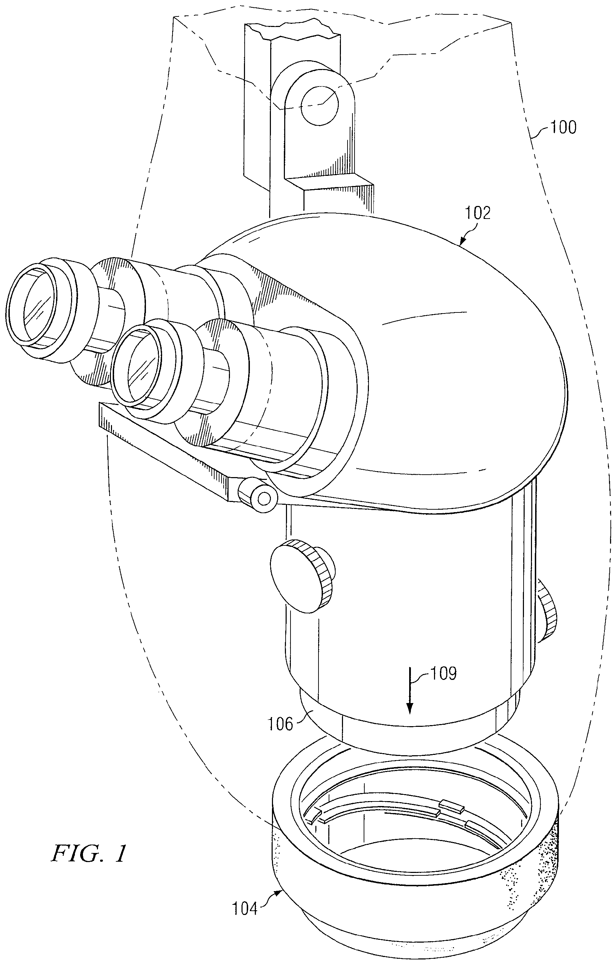

Example embodiments of the present invention and their advantages are best understood by referring now to FIGURES 1 through 4 of the drawings, in which like numerals refer to like parts.

FIGURE 1 is a perspective view of a microscope drape 100 coupled to a microscope 102 using a drape lens cover system 104. Although the present invention contemplates any suitable medical instrument being protected by drape 100, the present invention is particularly suitable for surgical microscopes, such as microscope 102 illustrated in FIGURE 1.

Drape 100 functions to generate a protective barrier between microscope 102 and its environment. For example, in an embodiment where microscope 102 is a surgical microscope, drape 100 protects microscope 102 from any bodily fluids, surgical fluids, and/or other materials during a surgical procedure from coming into contact with microscope 102. Conversely, any contaminants associated with microscope 102 are prevented from coming into contact with a patient during a surgical procedure. Any suitable drape 100 formed from any suitable material is contemplated by the present invention to cover microscope 102.

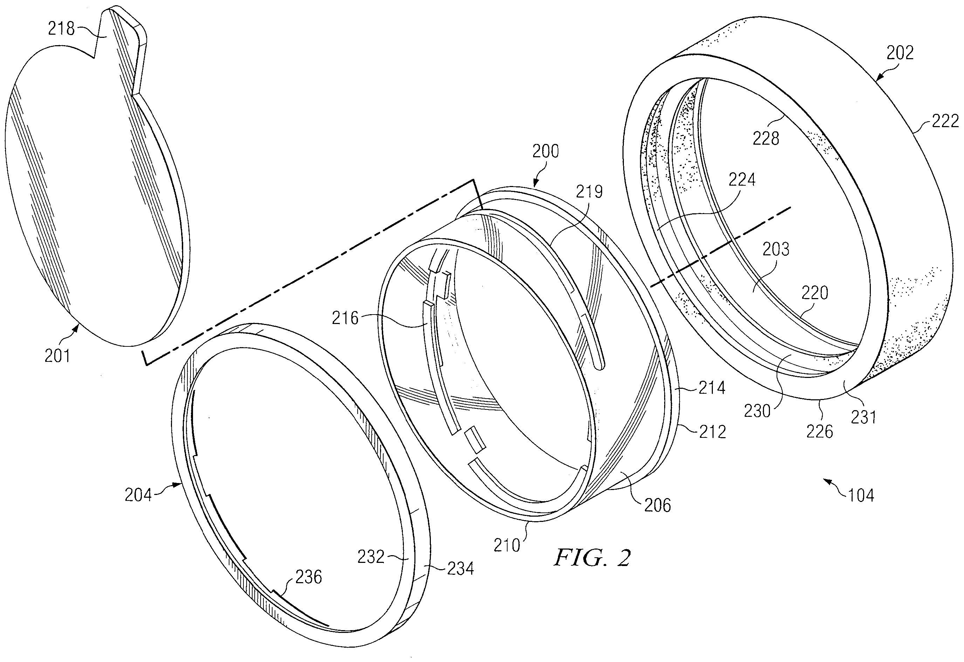

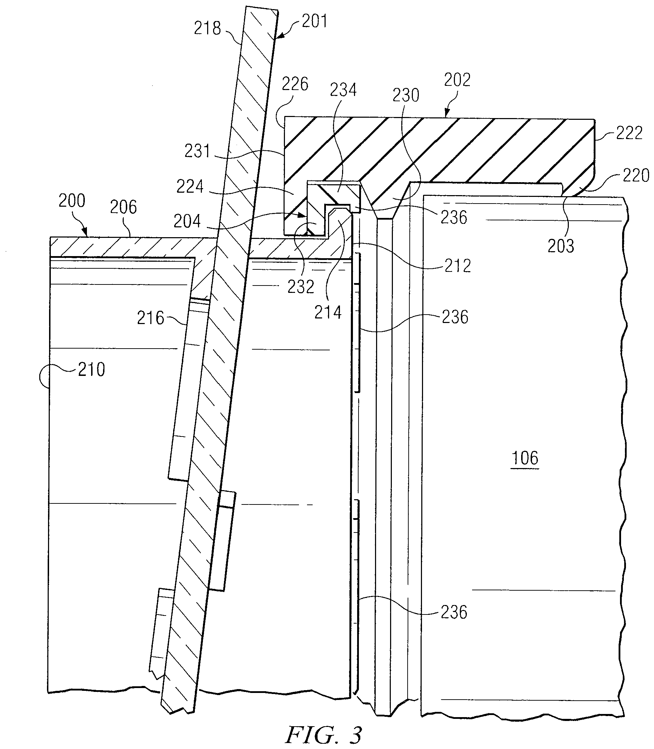

In the illustrated embodiment, drape lens cover system 104 couples to an objective lens barrel 106 of microscope 102; however, drape lens cover system 104 may couple to other portions of microscope 102 within the teachings of the present invention. Because there are many different types of microscopes available in the medical industry, many different sizes of objective lens barrels are encountered. Thus, a microscope drape lens cover system that fits the objective lens barrel of one microscope may not fit the objective lens barrel of another microscope. In addition, in order to be able to protect the objective lens of a microscope and still be able to see a surgical area, a transparent protective lens is typically associated with the drape lens cover system. The transparent protective lens, depending on its positioning, may cause an undesirable glare to the user of the microscope, which may complicate the surgical procedure. This transparent protective lens may also encounter bodily and/or surgical fluids during a surgical procedure, which means that the lens may need to be replaced during the surgical procedure. The present invention addresses these problems, and others, by providing drape lens cover system 104, as described in more detail below in conjunction with FIGURES 2 and 3.

FIGURE 2 is an exploded, perspective view of microscope drape lens cover system 104 according to one embodiment of the present invention. The components are disassembled in order to reveal other components and features that are not visible when the drape lens cover system 104 is assembled. In the illustrated embodiment, drape lens cover system 104 includes a housing 200 having a removable transparent protective lens 201, an objective lens barrel coupling member 202 having an objective lens barrel aperture 203, and a fitting or track system 204 that is positioned between the housing 200 and the objective lens barrel coupling member 202. An advantage of the illustrated embodiment is that housing 200 is rotatably secured in objective lens barrel coupling member 202 by an outwardly extending flange 214 of housing 200 that lies adjacent to an inwardly extending retainer flange 224 formed on the objective lens barrel coupling member 202. When assembled, inwardly extending retainer flange 224 is configured to maintain outwardly extending flange 214 within the objective lens barrel coupling member 202. Fitting 204 secures lens housing 200 in objective lens barrel coupling member 202 and allows housing 200 to rotate with respect to objective lens barrel coupling member 202 and objective lens barrel 106.

As described in more detail below in conjunction with FIGURE 3, housing 200 is rotatably coupled to objective lens barrel coupling member 202 in order to facilitate the rotation of transparent protective lens 201 to substantially reduce or eliminate any glare during a surgical procedure. Housing 200 may be any suitable size and shape and may be formed from any suitable material. In a particular embodiment, housing 200 may be made of plastic. Specific materials that may be used to form housing 200 include polyethylene, acrylonitrile butadiene styrene (ABS), nylon, or other similar type materials.

In the illustrated embodiment, housing 200 includes a cylindrical body portion 206 that forms an annular surface with a first edge 210 and a second edge 212. Housing 200 also includes an outwardly extending flange 214 disposed proximate second edge 212 and around a perimeter of housing 200. As such, outwardly extending flange 214 forms a lip that extends from body portion 206. As will be described in more detail below, outwardly extending flange 214 is configured to secure housing 200 within fitting 204.

Housing 200 functions to house transparent protective lens 201. In particular embodiments, lens 201 may be made of glass, acrylic, Polystyrene, Polycarbonate, Polymethylmethacrylate (PMMA), and Co-Polyester. These materials are provided as examples only, however. It is recognized that lens 201 may be formed from any suitable transparent material. It is further recognized that these materials and other suitable materials may be uncoated. Alternatively, the materials may be coated with an-antireflective coating to reduce glare.

Lens 201 may be coupled within housing 200 in any suitable manner. In one embodiment, a plurality of inwardly protruding tabs 216 are utilized to secure lens 201 therein. Inwardly protruding tabs 216 are integrally formed on the interior surface of body portion 206 and are described in more detail with respect to FIGURE 3. In the illustration of FIGURE 2, however, it can be seen that inwardly protruding tabs 216 are disposed at an angle. As a result, when transparent protective lens 201 is disposed on or between inwardly protruding tabs 216, transparent protective lens 201 is oriented at an angle. Specifically, transparent protective lens 201 is oriented such that a geometric normal to the lens 201 forms an angle with respect to an optical axis 109 of an objective lens housed within the objective lens barrel 106 when the objective lens barrel coupling member 202 is coupled to the objective lens barrel 106. Although any suitable angle may be utilized for lens 201, the angling of lens 201 facilitates the substantial reduction or elimination of any glare encountered during a surgical procedure. Depending on the lighting within an operating room, a glare may occur to a user of microscope 102. In order to reduce or eliminate that glare, the user merely rotates housing 200 to change the angle of refraction of the light so that it does not shine into the objective lens of microscope 102 in an undesirable manner.

In one embodiment, lens 201 includes a tab 218 for facilitating removal of transparent protective lens 201 from housing 200 in the event that lens 201 becomes damaged or unusable during a surgical procedure. Tab 218 is formed integral to and from the same material as lens 201 and enables a user of microscope 102 to handle lens 201 without dirtying or smudging the portions of lens 201 that are viewed through microscope 102. To insert lens 201 within housing 200, body portion 206 includes a slot 219. Lens 201 may be inserted into slot 214 such that lens 201 is positioned between inwardly protruding tabs 216. When lens 201 is disposed within housing 200, tab 218 may extend past the outside perimeter of housing 200 such that lens 210 may be easily removed and replaced without requiring that drape lens cover system 104 or housing 200 be removed from microscope 102. Tab 218 may also help facilitate the rotating of housing 200 with respect to objective lens barrel 106.

Objective lens barrel coupling member 202 functions to couple housing 200 to objective lens barrel 106 of microscope 102. Coupling member 202 is a cylindrical ring formed out of any appropriate material and is generally of a diameter that is slightly larger than the diameter of objective lens barrel 106. In a particular embodiment, coupling member 202 may be formed out of elastomer or another flexible material. For frictionally retaining flexible coupling member 202 around objective lens barrel 106, flexible coupling member 202 includes a first retainer flange 220 formed around the perimeter of the interior side of flexible coupling member 202. First retainer flange 220 is formed proximate a first edge 222 which is disposed proximate objective lens barrel 106 when drape lens cover system 104 is coupled to microscope 102. First retainer flange 220 defines objective lens barrel aperture 203, which has a diameter slightly smaller than the diameter of objective lens barrel 106. However, when flexible coupling member 202 is made of a flexible material such as elastomer, flexible coupling member 202 may be stretched over the end of objective lens barrel 106 and first retainer flange 220 may elastically constrict about objective lens barrel 106. Accordingly, first retainer flange 220 holds objective lens barrel coupling member 202 in place around objective lens barrel 106. Although objective lens barrel aperture 203 is illustrated as being circular, it is recognized that objective lens barrel aperture 203 may have any suitable size and shape. Other suitable shapes are contemplated by the present invention.

In the illustrated embodiment, flexible coupling member 202 includes a second retainer flange 224 for retaining outwardly extending flange 214 of housing 200. Second retainer flange 224 is formed around the perimeter of the interior side of flexible coupling member 202 proximate a second edge 226, which is disposed proximate housing 200 when housing 200 is coupled to flexible coupling member 202. Second retainer flange 224 defines a housing aperture 228, which has a diameter substantially the same as the diameter of housing 200. Second retainer flange 224 forms a ledge upon which outwardly extending flange 214 of housing 200 rests when housing 200 is coupled to flexible coupling member 202. Although housing aperture 228 is illustrated as being circular, it is recognized that housing aperture 228 may have any suitable size and shape corresponding with the size and shape of housing 200.

As illustrated, coupling member 202 includes a third retainer flange 230. Third retainer flange 230 is formed around the perimeter of the interior side of flexible coupling member 202. Third retainer flange 230 may be formed at any location within the interior surface of flexible coupling member 202. In a particular embodiment, third retainer flange 230 may be formed at a midpoint location between first edge 222 and second edge 226. Third retainer flange 230 functions to separate objective lens barrel 106 from housing 200 within flexible coupling member 202. Thus, where flexible coupling member 202 includes third retainer flange 230, outwardly extending flange 214 of housing 200 may be nested between second retainer flange 224 and third retainer flange 230. By contrast, the edge of objective lens barrel 106 may next between first retainer flange 220 and second retainer flange 224.

Objective lens barrel coupling member 202 also functions to couple drape lens cover system 104 to drape 100 via an annular surface 231 disposed around a perimeter of objective lens barrel coupling member 202 proximate second edge 226. Drape 100 may be coupled to annular surface 231 in any suitable manner, such as adhesive coupling.

As described above, drape lens cover system 104 includes a fitting 204 disposed between housing 200 and flexible coupling member 202 for facilitating the rotation of housing 200 (and, thus, lens 201 within housing) with respect to objective lens barrel 106. Fitting 204 is a substantially L-shaped member having two legs. Fitting 204 may be slid down the annular surface 206 of housing 200 from first edge 210 to second edge 212. Example materials that may be used to form fitting 204 include polyethylene, acrylonitrile butadiene styrene (ABS), nylon, or other similar type materials. First and second legs 232 and 234 of fitting 204 are disposed between outwardly extending flange 214 of housing 200 and second retainer flange 224 of flexible coupling member 202. Friction prevents fitting 204 from rotating with respect to flexible coupling member 202. However, fitting 204 reduces friction between housing 200 and flexible coupling member 202 and enables housing 200 to be easily rotated within flexible coupling member 202. Thus, in operation, flexible coupling member 202 is frictionally retained about objective lens barrel 106 and is not rotatable with respect to objective lens barrel 106. Similarly, fitting 204 is frictionally retained within second retainer flange 224 of flexible coupling member 202 and may not be rotated with respect to flexible coupling member 202 and/or objective lens barrel 106. However, housing 200 is rotatable with respect to objective lens barrel 106, fitting 204 and flexible coupling member 202.

In the illustrated embodiment, fitting 204 includes a plurality of tabs 236 extending from first leg 232. Tabs 236 function to secure fitting 204 on housing 200. Tabs 236 extend over the upper edge of outwardly extending flange 214 and prevent fitting 204 from sliding down the cylindrical body portion 206 of housing 200. Additionally, tabs 236 further reduce friction that may prevent or hinder the rotation of housing 200 with respect to flexible coupling member 202. Stated differently, tabs 236 may reduce the effort required to rotate housing 200 within flexible coupling member 202. Although any suitable number of tabs may be utilized, it is generally recognized that tabs 236 are optional and may be omitted. Alternatively, tabs 236 may be replaced with a continuous annular element having any suitable dimensions.

FIGURE 3 is a partial cross-sectional view showing the arrangement of the components of drape lens cover system 104 when assembled to objective lens barrel 106. Specifically, FIGURE 3 illustrates the assembly of housing 200, objective lens barrel coupling member 202, fitting 204, and transparent protective lens 201 when assembled together and upon the objective lens barrel 106.

As can be seen in the illustrated embodiment, fitting 204 lies adjacent outwardly extending flange 214 such that fitting 204 is secured between second retainer flange 224 and outwardly extending flange 214. Fitting 204 is secured in an inner depression formed in objective lens barrel coupling member 202 between second retainer flange 224 and third retainer flange 230. Friction between flexible coupling member 202 (second retainer flange 224, specifically) and fitting 204 holds fitting 204 securely in place and prevents rotation of fitting 204. By contrast, fitting 204 functions as a track in which outwardly extending flange 214 may easily rotate. Thus, housing 200 is rotatably secured adjacent first leg 232 and second leg 234 of fitting 204. Locking tab 236 prevents outwardly extending flange 214 from slipping outside of the track created by first leg 232 and second leg 234.

The materials selected for the various components illustrated in FIGURE 3 may provide for the functionality described above. For example, in a particular embodiment, lens housing 200 and fitting 204 may be formed of a relatively rigid material, such as plastic. Specific materials that may be used include polyethylene, acrylonitrile butadiene styrene (ABS), nylon, or other similar type materials. Lens housing 200 and fitting 204 are made of relatively rigid material in order to provide a relatively rigid structure for transparent protective lens 201 and to allow the outwardly extending flange 214 to slide freely relative to fitting 204. By contrast, objective lens barrel coupling member 202 may be formed of a elastomer or another flexible material such that inwardly protruding first retainer flange 220 of objective lens barrel coupling member 202 is configured to provide a resilient friction fit to the objective lens barrel 106. Specifically, first retainer flange 220 and/or the objective lens barrel coupling member 202 may be deformed slightly when placed over objective lens barrel with a resilient friction fit. A flexible material such as elastomer also allows for the creation of friction between the flexible coupling member 202 and fitting 204 to prevent fitting 204 from rotating within flexible coupling member 202.

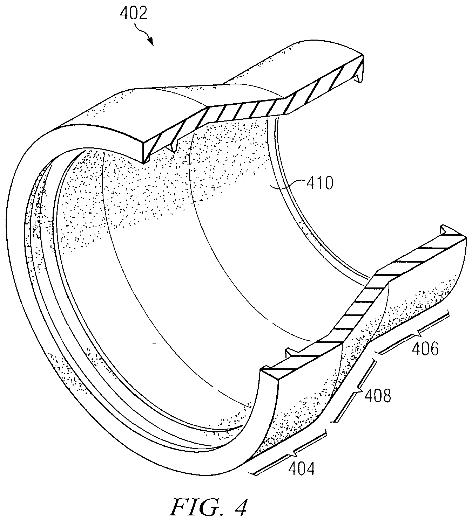

FIGURE 4 is a cross-sectional view illustrating a coupling member 402 in accordance in accordance with an alternative embodiment. Although coupling member 402 is similar to coupling member 202 of FIGURE 3, objective lens barrel coupling member 402 is configured to couple to an objective lens barrel that has a diameter smaller than that which is coupled to coupling member 202.

In the illustrated embodiment, coupling member 402 includes a first portion 404, a second portion 406, and a third portion 408. Each of the first, second and third portions include generally cylindrical annular surfaces. However, first portion 404 has an exterior diameter that is larger than the exterior diameter of second portion 406. In particular embodiments, the exterior diameter of first portion 404 is sufficient to define a housing aperture of an appropriate diameter for coupling to housing 200 of FIGURE 1. Second portion 406 defines an interior objective lens barrel aperture 410 that is approximately the same size and shape of an objective lens barrel 106. Although a generally circular shape of objective lens barrel aperture 410 is illustrated in FIGURE 4, other suitable shapes are contemplated by the present invention. Objective lens barrel aperture 410 has a diameter slightly larger than the diameter of an objective lens barrel to which it attaches (not illustrated). However, because the diameter of second portion 406 is smaller, the diameter of objective lens barrel aperture 410 may also be smaller. Accordingly, in particular embodiments, a smaller objective lens barrel may be accommodated by objective lens barrel aperture 410.

In the illustrated embodiment, coupling member 402 also includes a third portion 408. Third portion 408 is a transitioning portion that couples first portion 404 to second portion 406. The exterior diameter of third portion 408 proximate first portion 404 is the same as the exterior diameter of first portion 404. Similarly, the exterior diameter of third portion 408 proximate second portion 406 is the same as the exterior diameter of second portion 406. Thus, the outside annular surface defined by third portion 408 slopes from a larger diameter proximate first portion 404 to a smaller diameter proximate second portion 406. Although a third portion 408 is illustrated, it is generally recognized that third portion 408 may be omitted. In such an embodiment, the diameter change between first portion 404 and second portion 406 will be depicted as a step rather than as a slope.

Although embodiments of the invention and some of their advantages are described in detail, a person skilled in the art could make various alterations, additions, and omissions without departing from the scope of the present invention as defined by the appended claims.

## Claims

1. A microscope drape coupling system, comprising:
a coupling member (202) configured to be placed around and to couple to an objective lens barrel (106) of a microscope (102), wherein said objective lens barrel (106) extends into a radial and into a longitudinal direction; and
a lens housing (200) coupled to the coupling member (202), the lens housing (200) having a transparent protective lens (201) disposed within the lens housing (200), the transparent protective lens (201) positioned such that a geometric normal of the transparent protective lens (201) forms an angle with respect to an optical axis of an objective lens housed within the objective lens barrel (106) when the housing (200) is coupled to the objective lens barrel (106); **characterised by** a fitting (204) disposed between the coupling member (202) and the lens housing (200) in the radial and the longitudinal direction, the fitting (204) frictioanally retained by the coupling member (202) such that the rotation of the fitting (204) relative to the coupling member (202) is prevented, the fitting (204) is further configured to reduce friction between the lens housing (200) and the flexible coupling member (202) and thereby enable rotation of the lens housing (200) with respect to the coupling member (202).

2. The microscope drape coupling system of claim 1, wherein the lens housing (200) has an outwardly extending flange (214) that lies adjacent an inwardly extending flange (224) of the objective lens barrel coupling member (202), the fitting (204) disposed between the outwardly extending flange (214) of the housing (200) and the inwardly extending flange (224) of the objective lens barrel coupling member (202).

3. The microscope drape coupling system of claim 1 or 2, wherein the fitting (204) is an L-shaped member comprising a first leg and a second leg.

4. The microscope drape coupling system according to any of claims 1, 2 or 3, wherein the coupling member (202) is of a flexible material, preferably a polymer of a thermoplastic elastomer.

5. The microscope drape coupling system according to any one of claims 1-4, wherein the lens housing (200) is of a relatively rigid material, preferably the relatively rigid material is selected from the group consisting of polycarbonate, polyethylene, acrylonitrile butadiene (ABS), and nylon; and/or
the microscope drape coupling system wherein the fitting (204) is of a relatively rigid material, preferably the relatively rigid material is selected from the group consisting of polycarbonate, polyethylene , acrylonitrile butadiene (ABS), and nylon.

6. The microscope drape coupling system according to any one of claims 1-5, wherein the transparent protective lens (201) is of a material selected from the group consisting of glass, acrylic, polystyrene, polycarbonate, polymethylmethacrylate (PMMA), and co-polyester, preferably the transparent protective lens is coated with an anti-reflective coating.

7. The microscope drape coupling system according to any one of claims 1-6, wherein the transparent protective lens (201) is removable form the housing, and/or the microscope drape coupling system wherein the transparent protective lens (201) comprises a tab for facilitating the removal and replacement of transparent protective lens (201) from the housing (200), and/or
the microscope drape coupling system wherein the coupling member (202) comprises an annular surface disposed around a perimeter of the coupling member, the annular surface configured to be coupled to a drape; and/or
the microscope drape coupling system wherein an exterior surface of the coupling member (202) comprises
a first portion of a first diameter, the first portion defining a housing aperture; and a second portion of a second diameter that is smaller than the first diameter, the second portion defining an objective lens barrel aperture; and
wherein the objective lens barrel aperture is smaller than the housing aperture.

8. A microscope drape system comprising:
the microscope drape coupling system of any of claims 1 to 7;
the microscope (102); and
a drape (100) positionable to provide a protective barrier
between the microscope and a surgical environment.

9. A method of coupling a drape to a microscope by using the microscope drape coupling system according to claim 2, comprising; coupling the transparent protective lens (201) to the housing (200) such that they are coupled as required by claim 1; and positioning the fitting (204) (204) between the coupling member (202) and the lens housing (200) such that it is positioned as required by claim 1.

10. The method of claim 9 further comprising:
positioning the fitting (204) between the outwardly extending flange (214) of the housing (200) and the inwardly extending flange (224) of the objective lens barrel coupling member (202) such that the fitting (204) is positioned as required by claims 2.

## Patentansprüche

1. Kupplungssystem für Mikroskopabdeckung, umfassend:
ein Kupplungselement (202), das konfiguriert ist, um um einen Objektivtubus (106) eines Mikroskops (102) herum angeordnet und an ihn gekuppelt zu werden, wobei der Objektivtubus (106) sich in einer radialen und in einer Längsrichtung erstreckt; und
ein Linsengehäuse (200), das an das Kupplungselement (202) gekuppelt ist, wobei das Linsengehäuse (200) eine transparente Schutzlinse (201) aufweist, die im Inneren des Linsengehäuses (200) angeordnet ist, und die transparente Schutzlinse (201) so positioniert ist, dass eine geometrische Normale der transparenten Schutzlinse (201) einen Winkel zu einer optischen Achse einer Objektivlinse bildet, die im Inneren des Objektivtubus (106) angeordnet ist, wenn das Gehäuse (200) an den Objektivtubus (106) gekuppelt ist;
**gekennzeichnet durch** ein Fitting (204), das zwischen dem Kupplungselement (202) und dem Linsengehäuse (200) in der radialen und der longitudinalen Richtung angeordnet ist, wobei das Fitting (204) von dem Kupplungselement (202) so reibschlüssig festgehalten wird, dass die Rotation des Fittings (204) in Bezug auf das Kupplungselement (202) verhindert wird, wobei das Fitting (204) ferner konfiguriert ist, um die Reibung zwischen dem Linsengehäuse (200) und dem flexiblen Kupplungselement (202) zu reduzieren und damit die Rotation des Linsengehäuses (200) in Bezug auf das Kupplungselement (202) zu ermöglichen.

2. Kupplungssystem für Mikroskopabdeckung nach Anspruch 1, wobei das Linsengehäuse (200) einen sich nach außen erstreckenden Flansch (214) aufweist, der an einen sich nach innen erstreckenden Flansch (224) des Kupplungselements (202) des Objektivtubus angrenzt, wobei das Fitting (204) zwischen dem sich nach außen erstreckenden Flansch (214) des Gehäuses (200) und dem sich nach innen erstreckenden Flansch (224) des Kupplungselements (202) des Objektivtubus angeordnet ist.

3. Kupplungssystem für Mikroskopabdeckung nach Anspruch 1 oder 2, wobei das Fitting (204) ein L-förmiges Element ist, das einen ersten Schenkel und einen zweiten Schenkel aufweist.

4. Kupplungssystem für Mikroskopabdeckung nach einem der Ansprüche 1, 2 oder 3, wobei das Kupplungselement (202) aus flexiblem Material, vorzugsweise einem Polymer eines thermoplastischen Elastomers, ist.

5. Kupplungssystem für Mikroskopabdeckung nach einem der Ansprüche 1 - 4, wobei das Linsengehäuse (200) aus einem relativ starren Material besteht, wobei das relativ starre Material vorzugsweise aus der Gruppe ausgewählt ist, die aus Polycarbonat, Polyethylen, Acrylonitril-Butadien (ABS) und Nylon besteht; und/oder das Kupplungssystem für Mikroskopabdeckung, bei dem das Fitting (204) aus einem relativ starren Material ist, wobei das relativ starre Material aus der Gruppe ausgewählt ist, die aus Polycarbonat, Polyethylen, Acrylonitril-Butadien (ABS) und Nylon besteht.

6. Kupplungssystem für Mikroskopabdeckung nach einem der Ansprüche 1 - 5, wobei die transparente Schutzlinse (201) aus einem Material ist, das aus der Gruppe ausgewählt ist, die aus Glas, Acryl, Polystyrol, Polycarbonat, Polymethylmethacrylat (PMMA) und Co-Polyester besteht, wobei vorzugsweise die transparente Schutzlinse mit einer Anti-Reflex-Schicht überzogen ist.

7. Kupplungssystem für Mikroskopabdeckung nach einem der Ansprüche 1 - 6, wobei die transparente Schutzlinse (201) vom Gehäuse entfernt werden kann; und bzw. oder Kupplungssystem für Mikroskopabdeckung, bei dem die transparente Schutzlinse (201) eine Lasche aufweist, um das Entfernen und Ersetzen der transparenten Schutzlinse (201) aus dem Gehäuse (200) zu erleichtern; und/oder Kupplungssystem für Mikroskopabdeckung, bei dem das Kupplungselement (202) eine ringförmige Fläche aufweist, die um einen Umfang des Kupplungselements angeordnet ist, wobei die ringförmige Fläche konfiguriert ist, um an eine Abdeckung gekuppelt zu werden; und bzw. oder Kupplungssystem für Mikroskopabdeckung, bei dem eine Außenfläche des Kupplungselements (202) einen ersten Abschnitt eines ersten Durchmessers umfasst, wobei der erste Abschnitt eine Gehäuseöffnung definiert; und
einen zweiten Abschnitt eines zweiten Durchmessers, umfasst, der kleiner als der erste Durchmesser ist, wobei der zweite Abschnitt eine Objektivtubusöffnung definiert; und wobei die Objektivtubusöffnung kleiner ist als die Gehäuseöffnung.

8. Mikroskopabdeckungssystem umfassend:
das Kupplungssystem für Mikroskopabdeckung nach einem der Ansprüche 1 bis 7;
das Mikroskop (102) und
eine Abdeckung (100), die positioniert werden kann, um eine Abschirmung zwischen dem Mikroskop und einer Operations-Umgebung bereitzustellen.

9. Verfahren zum Kuppeln einer Abdeckung an ein Mikroskop durch Anwenden des Kupplungssystems für Mikroskopabdeckung nach Anspruch 2, umfassend:
Kuppeln der transparenten Schutzlinse (201) an das Gehäuse (200) derart, dass sie gemäß Anspruch 1 gekuppelt sind, und
Positionieren des Fittings (204) zwischen dem Kupplungselement (202) und dem Linsengehäuse (200) derart, dass es gemäß Anspruch 1 positioniert ist.

10. Verfahren nach Anspruch 9, ferner umfassend:
Positionieren des Fittings (204) zwischen dem sich nach außen erstreckenden Flansch (214) des Gehäuses (200) und dem sich nach innen erstreckenden Flansch (224) des Kupplungselements (202) für Objektivtubus derart, dass das Fitting (204) gemäß Anspruch 2 positioniert ist.

## Revendications

1. Système de couplage de rideau à un microscope, comprenant :
un élément de couplage (202) conçu pour être placé autour d'un barillet de lentille d'objectif (106) d'un microscope (102) et pour s'y coupler, le barillet de lentille d'objectif (106) s'étendant dans une direction radiale et longitudinale ; et
un boîtier pour lentille (200) couplé à l'élément de couplage (202), le boîtier pour lentille (200) comportant une lentille protectrice transparente (201) disposée dans le boîtier pour lentille (200), la lentille protectrice transparente (201) étant positionnée de sorte qu'une géométrie normale à la lentille protectrice transparente (201) forme un angle par rapport à un axe optique d'une lentille d'objectif logée dans le barillet de lentille d'objectif (106) quand le boîtier (200) est couplé au barillet de lentille d'objectif (106) ; **caractérisé par** une monture (204) disposée entre l'élément de couplage (202) et le boîtier pour lentille (200) dans la direction radiale et longitudinale, la monture (204) étant retenue par frottement par l'élément de couplage (202) de sorte que la rotation de la monture (204) par rapport à l'élément de couplage (202) est empêchée, la monture (204) étant en outre conçue pour réduire le frottement entre le boîtier pour lentille (200) et l'élément de couplage flexible (202), ce qui permet la rotation du boîtier pour lentille (200) par rapport à l'élément de couplage (202).

2. Système de couplage de rideau à un microscope selon la revendication 1, dans lequel le boîtier pour lentille (200) comporte une bride à extension extérieure (214) qui repose à côté d'une bride à extension intérieure (224) de l'élément de couplage (202) de barillet de lentille d'objectif, la monture (204) disposée entre la bride à extension extérieure (214) du boîtier (200) et la bride à extension intérieure (224) du barillet de l'élément de couplage (202) de barillet de lentille d'objectif.

3. Système de couplage de rideau à un microscope selon la revendication 1 ou 2, dans lequel la monture (204) est un élément en forme de L comprenant un premier jambage et un second jambage.

4. Système de couplage de rideau à un microscope selon l'une quelconque des revendications 1, 2 ou 3, dans lequel l'élément de couplage (202) est en matériau flexible, de préférence en polymère d'un élastomère thermoplastique.

5. Système de couplage de rideau à un microscope selon l'une quelconque des revendications 1 à 4, dans lequel le boîtier pour lentille (200) est en matériau relativement rigide, le matériau relativement rigide étant de préférence choisi dans l'ensemble constitué de polycarbonate, de polyéthylène, de butadiène-acrylonitrile (ABS) et de nylon ; et/ou
système de couplage de rideau à un microscope dans lequel la monture (204) est en matériau relativement rigide, le matériau relativement rigide étant de préférence choisi dans l'ensemble constitué de polycarbonate, de polyéthylène, de butadiène-acrylonitrile (ABS) et de nylon.

6. Système de couplage de rideau à un microscope selon l'une quelconque des revendications 1 à 5, dans lequel la lentille protectrice transparente (201) est en matériau choisi dans l'ensemble constitué de verre, d'acrylique, de polystyrène, de polycarbonate, de poly(méthacrylate de méthyle) (PMMA) et de copolyester, la lentille protectrice transparente étant de préférence revêtue d'un revêtement antiréfléchissant.

7. Système de couplage de rideau à un microscope selon l'une quelconque des revendications 1 à 6, dans lequel la lentille protectrice transparente (201) est amovible du boîtier, et/ou système de couplage de rideau à un microscope dans lequel la lentille protectrice transparente (201) comprend une patte servant à faciliter la dépose et le remplacement de la lentille protectrice transparente (201) du boîtier (200) ; et/ou système de couplage de rideau à un microscope dans lequel l'élément de couplage (202) comprend une surface annulaire disposée autour d'un périmètre de l'élément de couplage, la surface annulaire étant conçue pour être couplée à un rideau ; et/ou système de couplage de rideau à un microscope dans lequel une surface extérieure de l'élément de couplage (202) comprend :
une première partie d'un premier diamètre, la première partie définissant une ouverture de boîtier ; et
une seconde partie d'un second diamètre qui est plus petit que le premier diamètre, la seconde partie définissant une ouverture de barillet de lentille d'objectif ; et
dans lequel l'ouverture de barillet de lentille d'objectif est plus petite que l'ouverture de boîtier.

8. Système de rideau de microscope comprenant :
le système de couplage de rideau à un microscope selon l'une quelconque des revendications 1 à 7 ;
le microscope (102) ; et
un rideau (100) positionnable pour obtenir une barrière protectrice entre le microscope et un environnement chirurgical.

9. Procédé de couplage d'un rideau à un microscope par usage du système de couplage de rideau à un microscope conforme à la revendication 2, comprenant le couplage de la lentille protectrice transparente (201) au boîtier (200) de sorte qu'ils sont couplés tel que requis par la revendication 1 ; et
le positionnement de la monture (204) entre l'élément de couplage (202) et le boîtier pour lentille (200) de sorte qu'elle est positionnée tel que requis par la revendication 1.

10. Procédé selon la revendication 9, comprenant en outre : le positionnement de la monture (204) entre la bride à extension extérieure (214) du boîtier (200) et la bride à extension intérieure (224) de l'élément de couplage (202) du barillet de lentille d'objectif, de sorte que la monture (204) est positionnée tel que requis par la revendication 2.
